# EUROPÄISCHE PATENTSCHRIFT

(11) **EP 0 871 407 B1**
(45) Veröffentlichungstag und Bekanntmachung des Hinweises auf die Patenterteilung: **29.03.2000**
(21) Anmeldenummer: 96942346.6
(22) Anmeldetag: 04.12.1996
(51) Int. Cl.: A61B 19/00, A61B 17/34

(54) **VORRICHTUNG ZUR ANSTEUERUNG KÖRPERLICHER STRUKTUREN**
DEVICE FOR CONTROLLING THREE-DIMENSIONAL STRUCTURES
DISPOSITIF POUR PILOTER DES STRUCTURES TRIDIMENSIONNELLES

(30) Priorität: 04.12.1995 DE 29519103 U
(43) Veröffentlichungstag der Anmeldung: 21.10.1998
(73) Patentinhaber: Vogele, Michael, 86830 Schwabmünchen (DE)
(72) Erfinder: Vogele, Michael, 86830 Schwabmünchen (DE)
(74) Vertreter: Fiener, Josef
(86) Internationale Anmeldenummer: EP9605418
(87) Internationale Veröffentlichungsnummer: WO9720515

(56) Entgegenhaltungen:
- WO-A-94/28819
- WO-A-95/07055
- US-A- 5 080 662
- US-A- 5 230 623
- US-A- 5 281 232
- US-A- 5 464 411

## Beschreibung

Die Erfindung betrifft eine Vorrichtung zur Ansteuerung körperlicher Strukturen, insbesondere zur Einführung von Punktionsnadeln oder Operationssonden gemäß den oberbegrifflichen Merkmalen des Anspruches 1. Eine derartige Vorrichtung ist aus der WO-A-95/07055 bekannt.

Bei zahlreichen operativen oder stereotaktischen Eingriffen ist die genaue Ansteuerung von Punkten am oder im Körper erforderlich. Vor allem durch die Einbeziehung moderner Computertechnologien wie Computer-Tomographie (CT) ist es möglich geworden, die erforderlichen Eintrittsorte, Eintrittstiefen und Eintrittsrichtungen der medizinischen Instrumente festzulegen. Somit muß auch eine Zielvorrichtung zur Führung dieser Instrumente ebenfalls den erhöhten Genauigkeitsansprüchen gerecht werden. Mittels der z. B. durch CT ermittelten Patientendaten und Parameter soll dann ein Instrument an den definierten Punkt am oder im Körper gebracht werden können, was bisher jedoch nur umständlich, zeitraubend und häufig patientenbelastend möglich war.

Folgende Faktoren sind dabei für den Einsatz einer zu diesem Zweck konzipierten Zielvorrichtung zur Führung medizinischer Instrumente entscheidend:
1. Hohe Zielgenauigkeit;
2. Einfache und schnelle Reproduzierbarkeit;
3. Hohe Variabilität;
4. Möglichkeit zur Einführung mehrerer Instrumente, parallel, oder in geringem Abstand zueinander, wobei für jedes Instrument die Eintrittstiefe individuell festgelegt werden kann;
5. Röntgendurchlässige Materialien;
6. Wiederholte Einbringung von verschiedenen Instrumenten, welche unterschiedliche Durchmesser und Formen aufweisen, an den jeweils gleichen Zielpunkt.

Die bisher in der Praxis verwendeten Zielvorrichtungen bestehen aus einer massiven Metallführungsröhre, welche an einen stereotaktischen Rahmen aus Metall-Bügeln oder -Bogen angebracht ist z. B. gemäß der US-A-52 57 998, US-A-51 76 689 oder US-A-52 01 742. Diese Vorrichtungen entsprechen vor allem nicht bzw. nicht voll den vorstehend genannten Erfordernissen, insbesondere aus den nachfolgenden Gründen.

Die bisher verwendeten Zielvorrichtungen sind oftmals nicht abnehmbar und wieder in exakt gleicher Weise anbringbar. Mit dem oft sehr hohen Gewicht des stereotaktischen Rahmens ist eine reproduzierbare Positionierung sehr schwierig. Um die Reproduzierbarkeit dennoch zu ermöglichen, sind oftmals Meßskalen angebracht, die jedoch schwierig und somit nur von eingeschultem Personal einzustellen sind. Die stereotaktische Genauigkeit leidet bei wiederholten Eingriffen oftmals darunter, da für jeden Patienten die Vorrichtung umgestellt werden muß. Da zudem die herkömmlichen Zielvorrichtungen an einen massiven Rahmen gebunden sind, ist oftmals die Variabilität eingeschränkt. Dies gilt sowohl für das Ansteuern oder Anfahren der unterschiedlichen Eintrittsorte mit der an diesem Rahmen aufgehängten Zielvorrichtung, als auch für den durch Zielvorrichtung und Rahmen beanspruchten Platz.

Nachfolgend soll die praktische Anwendung derartiger Vorrichtungen am Beispiel der CAS (computer assisted surgery) dargestellt werden. Die CAS stellt eine intraoperative Navigationshilfe dar. Vor dem stereotaktischen Eingriff wird der Patient im CT oder im MRI gescannt. Anschließend werden die einzelnen Körperschnitte des Patienten zu einem 3D-Objekt mit entsprechenden stereotaktischen Raumkoordinaten am Bearbeitungscomputer rekonstruiert und auf einen Monitor im Operationssaal transferiert. Dieses virtuelle Bild wird im Operationssaal auf den Patienten mit Hilfe eines mit dem Monitor gekoppelten, passiven mechanischen Armes, an dessen Ende sich eine Sonde befindet, geeicht. Vor dieser Patienteneichung ist es jedoch notwendig, die Sonde zu kalibrieren. Dabei wird die Sonde in einen Metallzylinder geführt und rotiert, wobei der Computer die genauen Daten (Länge, Form) der Sonde registriert. Dieser Schritt ist nicht zu vernachlässigen, da kleine Änderungen der Länge und der Form der Sonde große Auswirkungen auf die Systemgenauigkeit haben. Die üblicherweise verwendete Sonde hat eine Länge von ca. 160 mm und einen Durchmesser von 4 mm, wobei von der Sondenspitze aus betrachtet, der vordere Teil auf 1,65 mm reduziert ist. Nach der Sondeneichung teilen Sensoren an jeder der sechs Rotationsachsen des mechanischen Armes dem Computer die relativen Winkel der Armteile zueinander mit, wodurch der Computer in der Lage ist, die stereotaktischen Koordinaten der Sondenspitze zu errechnen. Die Patienteneichung erfolgt durch Anfahren mehrerer Punkte, z. B. anatomisch signifikante Punkte oder durch röntgendichte Eichpunkte (Marker) gekennzeichnete Punkte am Patienten bzw. an der Eichvorrichtung. Nach entsprechender Korrelation zum rekonstruierten 3D-Objekt am Bildschirm ist es dem Computer möglich, dieses 3D-Objekt in diesen virtuellen Raum einzupassen. Je besser die angefahrenen Punkte mit dem am Bildschirm korrelierten Punkten übereinstimmen, desto genauer stimmen virtuelles Bild und Patientenkörperteil überein, was zu einer hohen Genauigkeit des Systems führt. Der Operateur kann sich während des Eingriffes mit Hilfe von rekonstruiertem 3D-Objekt und mehreren zweidimensionalen Bildern, die immer die Sondenspitze anzeigen, orientieren.

Aus derartigen, in den letzten Jahren entwickelten Navigationssystemen und Verwendung dieser im Zusammenhang mit stereotaktischen Halterungen mit integrierten Eichvorrichtungen (z. B. einer Kopfhalterung gemäß DE-U-295 08 277 oder einer Oberkiefer-Hals/Schulterhalterung gemäß DE-U-295 11 995) ergeben sich die obigen Anforderungen an stereotaktische Zielvorrichtungen.

Im weiteren soll noch ein spezielles Anwendungsbeispiel im Zusammenhang mit der Strahlen-Therapie aufgeführt werden, wobei der praktische Ablauf eines Bestrahlungseinsatzes geschildert wird, bei welchem ein Navigationssystem eine stereotaktische Kopfhalterung und eine Zielvorrichtung Anwendung finden. Bei der Strahlen-Therapie werden Punktionsnadeln (sog. Pins) direkt in das zu bestrahlende Tumorgewebe vorgeschoben und anschließend erfolgt eine direkte Bestrahlung des Tumors durch radioaktive Substanzen, ausgehend von der Nadelspitze. Es gilt in diesem Fall, wenigstens eine Nadel exakt an einen Punkt (z. B. Tumormitte) vorzuschieben und dabei vitale Strukturen zu umgehen und zu schützen. Es sei betont, daß vor dem Einsatz computergestützer Navigationssysteme auf diesem Gebiet der Tumor bestmöglich per Hand ausgetastet wurde, die Nadel dann blind vorgeschoben und teilweise noch anschließend die Lage der Nadelspitze mittels CT oder MRI kontrolliert wurde.

Die Nachteile dieser Methode liegen auf der Hand: Der Tumor wurde oftmals verfehlt und eine Verletzung vitaler Strukturen verursacht. Durch den Einsatz computergestützer Navigationssysteme ist es zwar möglich geworden, entscheidende Verbesserungen auf diesem Gebiet zu erzielen, da auf dem Bildschirm anstatt der Lage der Sondenspitze nun die Lage der Nadelspitze im oder am Körper angezeigt wird. Dabei wird jedoch die Nadel noch weitgehend freihändig geführt und vorgeschoben und keine genaue Zielvorrichtung verwendet. Jedoch ergeben sich dabei zahlreiche Probleme:
a) Die Prozedur direkt am Patienten freihändig durchzuführen ist sehr zeitaufwendig, da dabei jedesmal sowohl der Eintrittspunkt aufgefunden und angesteuert werden muß, als auch die Vorschubrichtung in allen drei Raumebenen exakt beibehalten werden muß. Daneben muß während des anschließenden Vorschiebens praktisch simultan das Bild auf dem Monitor und die mit der Hand geführte Nadel im Auge behalten werden. Selbst für einen geübten Bediener ist dies sehr schwierig, man denke dabei nur an kleine unbewußte Handbewegungen. Aus diesem Grund muß die Nadel oftmals wieder zurückgezogen und korrigiert werden. Sowohl der hohe Zeitaufwand, als auch die Korrektur sind patientenbelastend.
b) Das Navigationssystem arbeitet auch nicht mit der gewohnten Genauigkeit, da die Nadel nicht annähernd die Steifigkeit der Sonde aufweist. Durch Manipulationen beim direkten Umgang mit Nadel kommt es oftmals zu minimalen Verbiegungen der Nadel. Da jedoch Verbiegungen der Nadel vom Computer nicht registriert oder berechnet werden können, liefert der Computer eine Fehlinformation über die momentane Lage der Nadelspitze im Raum, was logischerweise fatale Folgen haben kann.
c) Das Vorschieben mehrerer Nadeln, wobei für jede Nadel ein eigener Eintrittspunkt, eine eigene Richtung und ein eigener Zielpunkt und eine anschließende individuelle Kontrolle durchgeführt wird, ist bisher unmöglich, da nur ein Arm für eine Nadel zur Verfügung stand. Das Wechseln der Nadeln, d.h. Vorschub von Nadel Nr.1, Demontage vom Arm, Einbau von Nadel Nr.2 usw. hat zwei Hauptnachteile: Zum einen ist dies ein weiterer Ungenauigkeitsfaktor, da minimale Abweichungen der Nadelform, ähnlich wie auch bei der Nadelverbiegung den Computer die tatsächliche Position falsch berechnen lassen. Die normalerweise beim Wechsel der Sonde/Nadel durchgeführte Eichung jeder verwendeten Nadel ist aus Zeitgründen nicht praktikabel. Zum anderen wäre beim Wechsel der Nadel deren Verschiebung möglich und die Lage der Nadelspitze könnte auch nicht mehr kontrolliert werden.

Demzufolge liegt der Erfindung die Aufgabe zugrunde, eine Vorrichtung zur Ansteuerung körperlicher Strukturen zu schaffen, die die vorstehenden Anforderungen erfüllt, insbesondere bei einem einfachen Aufbau eine präzise, reproduzierbare und variable Führung für medizinische Instrumente zu schaffen.

Diese Aufgabe wird gelöst durch eine Vorrichtung mit den Merkmalen des Anspruches 1.

Durch die Anbringung von zwei winklig, bevorzugt in einer Ebene zu der Halte- und Zieleinrichtung verlaufende Schwenkachsen ist eine beliebige räumliche Positionierung und Ausrichtung der Halte- und Zieleinrichtung möglich. Dabei sind die Schwenkachsen wechselweise in Lagerelementen am Ende der Haltestäbe verschwenkbar und verschiebbar angeordnet, so daß bei Arretierung der einen Schwenkachse die Halte- und Zieleinrichtung um die andere Schwenkachse verstellt werden kann, so daß eine zielgerichtete Einstellung der Halte- und Zieleinrichtung in einer Raumachse bei Beibehaltung der anderen Raumachsen möglich ist. Damit ist eine sehr präzise Anpeilung von vorher beispielsweise im CT festgelegten Eingriffsorten möglich, so daß eine wesentliche Erleichterung der in der Neurochirurgie üblichen Eingriffe durch die exakte Führung von Instrumenten, insbesondere Punktionsnadeln oder Operationssonden erzielt wird.

Bevorzugte Ausführungsbeispiele sind Gegenstand der Unteransprüche. Zweckmäßig ist hierbei insbesondere die Ausrichtung der Schwenkachsen in einem rechten Winkel zueinander, da hierdurch die Raumachsen im Koordinatensystem, ähnlich wie bei den bildgebenden Verfahren als Referenzebenen gelegt werden können. Die Halte- und Zieleinrichtung weist hierbei insbesondere eine Vielzahl von rasterförmig angeordneten Führungsbohrungen in einem Würfel auf, um mehrere Instrumente gleichzeitig zum Einsatz zu bringen. Dabei können auch mehr als zwei Schwenkachsen vorgesehen sein.

Diese würfelförmige Halte- und Zieleinrichtung ermöglicht somit das Einführen mehrerer Instrumente nebeneinander, wobei durch den Bohrlochabstand und die Bohrungsrichtung sowohl der Abstand der einzubringenden Nadeln zueinander als auch deren Richtung festgelegt ist. Es können auch mehrere Halte- und Zieleinrichtungselemente z. B. durch Winkel oder Schienen zusammengefügt und damit die Zahl der Bohrungen und somit die Möglichkeit zum Einführen mehrerer Instrumente erhöht werden. Die Halte- und Zieleinrichtungselemente können aber auch jeweils an weiteren Haltestäben befestigt werden. Neben der Erhöhung der Zahl der Bohrungen ist sowohl durch die separate als auch durch kombinierte Aufhängung der Halte- und Zieleinrichtungselemente eine beliebige Lageeinstellung einzelner Halte- und Zieleinrichtungen zueinander möglich. Den angedeuteten Variationsmöglichkeiten sind im Rahmen der klinischen Erfordernisse praktisch keine Grenzen gesetzt.

Ein weiterer Vorteil der nach dem Baukastenprinzip beliebig erweiterbaren Halte- und Zieleinrichtung ist die Anbringung von Markierungen, mit deren Hilfe die Eindringtiefe optisch festgelegt und kontrolliert werden kann. Neben dieser Möglichkeit die Eindringtiefe optisch zu kontrollieren, erlaubt die Ausbildung der Halte- und Zieleinrichtung aus röntgendurchlässigen Material, z. B. Acrylglas die Kontrolle der Lage nach Einführung der Nadel mittels CT- oder MRI-Aufnahme. Nachdem das Hauptinstrument z. B. auf Tumormitte richtig eingestellt ist, können verschiedene Instrumente auf gleiche Art und Weise genau eingebracht werden. Dafür können auch Einlagerohre in dem "Zielwürfel" ausgetauscht werden, z. B. zur Einbringung von Nadeln Einlagerohre mit einem runden Innendurchmesser von wenigen mm, während durch die Wahl unrunder Innenausnehmungen verhindert wird, daß das eingebrachte Instrument sich unbeabsichtigt dreht. Dies ist z. B. beim mehrmaligen Einführen eines Endoskopes sinnvoll, wobei im eingeführten Zustand immer die exakt gleichen Bildaufnahmerichtungen geliefert werden, da die Eindringtiefe festgelegt ist und jede Rotation ausgeschaltet ist.

Die vorgeschlagene Zielvorrichtung mit den oben aufgeführten Vorteilen wurde speziell für CAS-Einsätze entwickelt und genügt im weitesten Sinne den eingangs genannten Erfordernissen. Der Patient wird dabei in einer stereotaktischen Kopfhalterung gescannt (z. B. mit Hilfe der Kopfhalterung nach dem Gebrauchsmuster DE-U-295 08 277 oder der Oberkiefer-Hals/Schulterhalterung gemäß Gebrauchsmuster DE-U-295 11 995). Dabei werden über eine Eichvorrichtung, die eindeutig in Relation zum Patientenkopf Punkte im Raum festlegt, Eichpunkte definiert. Bei der Kopfhalterung (Gebrauchsmuster DE-U-295 08 277) oder der Oberkiefer-Hals/Schulterhalterung (Gebrauchsmuster DE-U-295 11 995) werden diese Eichpunkte über eine an dem Oberkiefer angebrachte Eichvorrichtung definiert. Nach Rekonstruktion des Patientenkopfes und der Eichpunkte, kann nun schon vor dem tatsächlichen Eingriff die Zielvorrichtung komplett vorjustiert werden. Somit wird eine Simulation durchgeführt, indem nach Eichung die Sondenspitze im Bereich des stereotaktischen Rahmens umhergeführt wird, wobei auf dem Monitor die Lage im oder am virtuellen Patientenkopf beobachtet werden kann.

Nachfolgend wird ein bevorzugtes Ausführungsbeispiel anhand der Zeichnungen beschrieben. Hierbei zeigen:
- Fig. 1: eine Seitenansicht einer Vorrichtung mit Grundplatte, zwei Basishaltern mit Grund- und Haltestäben, die mit Kugelgelenken verbunden sind;
- Fig. 2: eine Draufsicht auf die Grundplatte, Basishalter mit Grund- und Haltestäben gemäß Fig. 1; und
- Fig. 3: eine Halte- und Zieleinrichtung in gesonderter Darstellung in Ansichtsrichtung gemäß Pfeil X in Fig. 2.

Eine aus nichtrostendem, magnetisierbarem Stahl gefertigte Grundplatte 1 ist an ihrer Unterseite mit einem Operationstisch 1a verbunden, z. B. mit Haltearme aus Metallklauen, die mit der Grundplatte 1 verschraubt sind. Die Grundplatte 1 kann gegenüber dem OP-Tisch la in horizontaler und in vertikaler Richtung verstellt und bis zu einem gewissen Grad um eine beliebige Achse gedreht werden, wobei in jeder Lage ein hohes Maß an Festigkeit gewährleistet ist. Die eine Halte- und Zieleinrichtung 10 tragenden Teile der Vorrichtung bestehen jeweils aus einem Basishalter 2, einem damit verbundenen, Grundstab 3 und einem mit diesem gelenkig verbundenen Haltestab 4, der am Ende jeweils ein Lagerelement 5 zur Befestigung der würfelförmigen Halte- und Zieleinrichtung 10 aufweist. Der Basishalter 2 kann an jeder Stelle der Grundplatte 1 mechanisch, magnetisch oder pneumatisch verankert werden. Die gelenkige Verbindung zwischen Grundstab 3 und Haltestab 4 ist jeweils mittels einer zentralen Arretierungsvorrichtung 12 in Form eines Handrades fixierbar. Bei einer bevorzugten hydraulischen Ausführung in Form eines Stativs wird die Kraftübertragung durch Rohre und damit die Lagefixierung jeweils zeitgleich auf die zwischen den Bauteilen 2 - 3, 3 - 4 und 4 - 5 vorgesehenen Kugelgelenke 23, 34 und 45 bewerkstelligt.

Als Führungsrohr für ein medizinisches Instrument 8 zur Ansteuerung eines Zielgewebes Z dient wenigstens eine Bohrung 9 in der Halte- und Zieleinrichtung 10. In die jeweilige Bohrung 9 kann auch eine Sonde oder ein Einlagerohr 11 zur Anpassung an das verwendete Instrument 8 eingebracht werden, insbesondere eine mit einem Anschlag zur Axialeinstellung versehene Punktionsnadel 8, wie dies hier dargestellt ist. Die Axialposition des Instrumentes 8 kann auch durch eine Klemmeinrichtung 17, hier in Form eines Schiebers exakt fixiert werden.

Zur Justierung der Vorrichtung werden die beiden Basishalter 2 mittels Formabschnitten 13, die auch eine seitliche Erweiterung der Grundplatte 1 erlauben, oder mittels Markierungen 14 vorpositioniert, dann eine Sonde in die Bohrung 9 eingeführt und anschließend die Sonde so lange im virtuellen Raum herumgeführt, bis die Sondenspitze am gewünschten Eintrittspunkt liegt und die Projektionslinie (= Verlängerung der Sondenspitze entlang der Sondenachse) deckungsgleich mit der Vorschubrichtung ist (welche sich am Bildschirm ablesbar zeigt). Dadurch wird der gewünschte Zielpunkt exakt angesteuert. Bei diesem Vorgang kommen die in den Unteransprüchen aufgeführten Zusatzeinrichtungen zum Tragen. Es sei an dieser Stelle insbesondere eine schematisch dargestellte Feintriebmechanik 15 zur Feineinstellung des Eintrittspunktes und der Eintrittsrichtung erwähnt. Je nach Bedarf können nach Einstellung der ersten Hauptführungsbohrung 9 weitere Bohrungen 9 individuell eingestellt werden und durch Anbau weiterer Halte- und Zieleinrichtungen 10 oder durch einen separaten Haltestab 4 mit Halte- und Zieleinrichtung 10 diese beliebig erweitert werden. Somit kann vorab durch eine Simulation (ohne Patienten!) die gesamte Halte- und Zieleinrichtung 10 in Relation zum stereotaktischen Rahmen und damit sowohl zur Eichvorrichtung als auch zum Patienten exakt eingestellt werden.

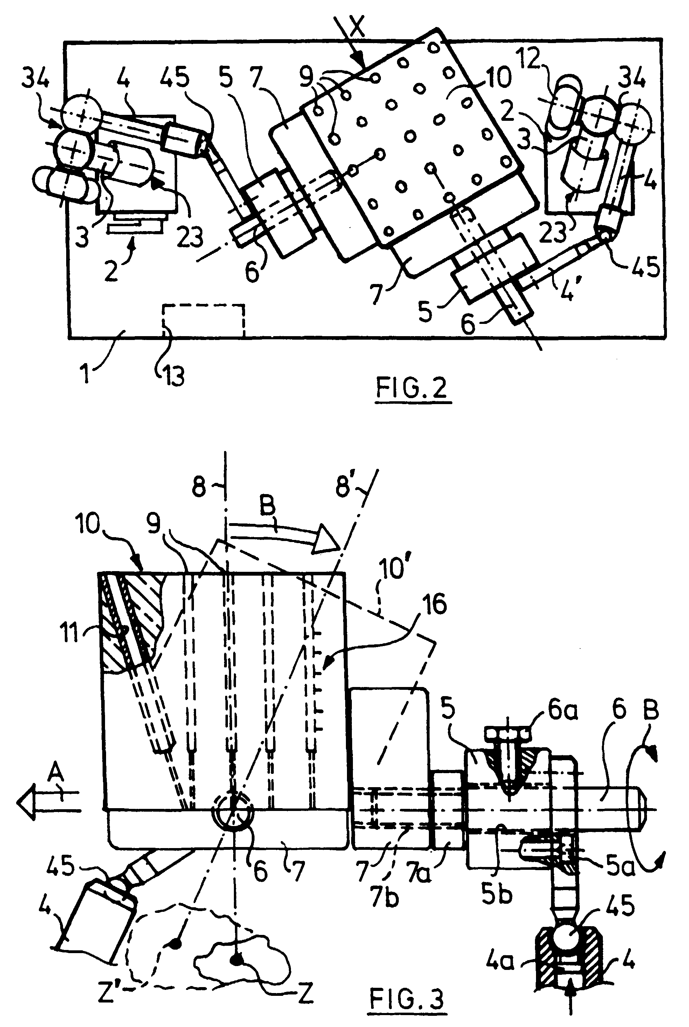

Beim Eingriff selbst wird nun der Patient auf exakt gleiche Art und Weise in den Rahmen eingepaßt, wie er im CT gescannt wurde. Es wird nun das Navigationssystem geeicht und die Halte- und Zieleinrichtung 10 auf stereotaktisch korrekte Weise angebracht. Zur Kontrolle kann nun nochmals eine Sonde in die bereits justierte Halte- und Zieleinrichtung 10 eingebracht, und wenn nötig, kann die Sonde nachjustiert werden. Die Nachjustierung ist, falls erforderlich, rasch durchführbar, da die Sonde durch die Vorjustierung schon beinahe die korrekte Lage hat. Zuletzt wird die Sonde herausgenommen und das passende Einlagerohr 11 in die Bohrung(-en) 9 eingepaßt. Die Bohrung 9 in dem Einlagerohr 11 entspricht exakt dem Außendurchmesser der einzuführenden Punktionsnadel 8, die anschließend eingeführt werden kann und nach definierter Eindringtiefe (am Bildschirm ablesbar) vorgeschoben und dann durch die Klemmeinrichtung 17 fixiert wird. Die tatsächliche Eindringtiefe der Nadel 8 wird entweder optisch durch eine Meßskala 16 (vgl. Fig. 3) oder durch einen Anschlag kontrolliert. Nach Erreichen des Zielpunktes Z mit der Nadelspitze kann somit die eigentliche Bestrahlung des Tumors beginnen. Nach einmaliger Simulation und Vorjustierung der Sonde kann der Eingriff am Patienten beliebig oft wiederholt werden.

Von wesentlicher Bedeutung hierbei ist die Lagerung der Halte- und Zieleinrichtung 10 mit den Führungsbohrungen 9 an zwei winklig zueinander ausgerichteten Schwenkachsen 6. Diese Schwenkachsen 6, die vorzugsweise aus Carbonwerkstoff hergestellt sind, werden bevorzugt rechtwinklig zueinander in einer Ebene (in Fig. 2= Zeichenebene) angeordnet. Die Schwenkachsen 6 sind hierbei in den beiden zugeordneten Lagerelementen 5 verschwenkbar, sowie auch verschiebbar angeordnet, wie dies durch die beiden Richtungspfeile B und A angedeutet ist. Das jeweilige zur würfelförmigen Halte- und Zieleinrichtung 10 hingelegene Ende der Schwenkachsen 6 ist dabei in einem Lagerblock 7 fixiert, insbesondere mittels eines Gewindes 7b und einer Kontermutter 7a, wie dies im Detail in Fig. 3 dargestellt ist. Die jeweilige Schwenkachse 6 ist in dem Lagerelement 5 in einer Bohrung 5b axial verschiebbar (Richtungspfeil A), sowie auch um die Rotationsachse (Richtungspfeil B) verdrehbar. In Fig. 3 ist diese Verschwenkbewegung um die senkrecht zur Zeichenebene ausgerichtete Schwenkachse 6 strichliert dargestellt, wobei die würfelförmige Halte- und Zieleinrichtung 10 hier nach rechts in die Position 10' geschwenkt wird. Dabei wird das in die Führungsbohrung 9 eingesteckte Instrument ebenfalls in die Position 8' mitverschwenkt, so daß von dem ursprünglich angepeilten Ziel Z nunmehr ein Ziel Z' angesteuert wird. Dieses Ziel Z' könnte ebenfalls angesteuert werden durch die etwas nach links versetzte Führungsbohrung 9 oder durch Axialverschiebung in Axialrichtung A. Hierzu könnte beispielsweise die Feststellschraube 6a in dem Lagerelement 5 gelöst werden und die Schwenkachse 6 nach links verschoben werden. Die Feststellschraube 6a könnte dabei auch ein Exzenter oder ähnlicher Mechanismus sein, so daß hierdurch ein Feinantrieb 15 verwirklicht wird, um eine millimetergenaue Feineinstellung des Zielortes Z zu erreichen.

Im Zusammenhang mit Fig. 3 sei darauf hingewiesen, daß das Schiebegelenk und die Schwenkachse 6 in den Lagerelementen 5 auch baulich getrennt ausgebildet sein können und die Bohrungen 9 auch radial verlaufend angeordnet sein können, wie dies für die hier linke Bohrung angedeutet ist. Im Teilschnitt ist hierbei auch die Anordnung eines Einlagerohres 11 dargestellt, wie dies zur Anpassung an das jeweils verwendete Instrument 8, beispielsweise einer relativ dünnen Punktionsnadel oder eine relativ dicken Sonde erforderlich ist.

Im rechten Teil der Fig. 3 ist die Halterung des Lagerelementes 5 näher dargestellt. Dieses Lagerelement 5 ist über einen Haltearm 4' und Befestigungselemente 5a, insbesondere Schrauben mit dem Kugelgelenk 45 des Haltestabes 4 verbunden. Das Kugelgelenk 45 ist dabei durch einen Stempel 4a arretierbar, indem die zentrale Arretiervorrichtung 12 mittels des Handrades betätigt wird und dabei der Stempel 4a gegen das Kugelgelenk 45 vorschiebt und somit arretiert.

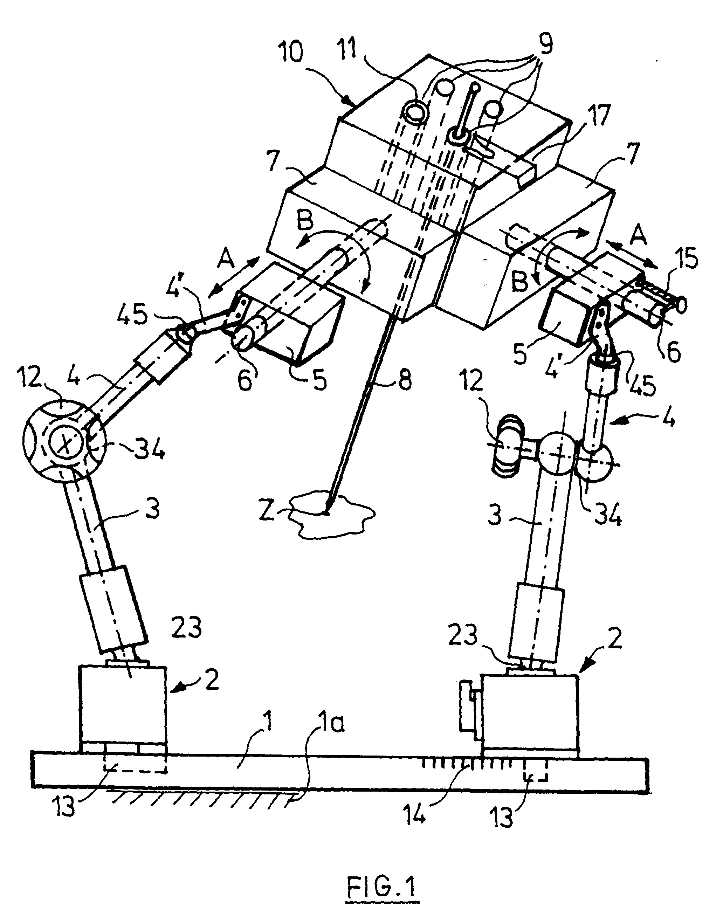

Es sei darauf hingewiesen, daß durch die Anordnung der Halte- und Zieleinrichung 10 an den beiden (oder mehr) Schwenkachsen 6 die räumliche Lage der Bohrungen 9 zur Führung der Instrumente 8 beliebig eingestellt werden kann. Wesentlich ist hierbei insbesondere, daß die Änderung der Justierung wechselweise erfolgt, d. h. eine zentrale Arretiereinrichtung 12 jeweils verriegelt bleibt, während die andere gelöst wird. Wenn beispielsweise die in Fig. 1 rechte Arretiervorrichtung 12 gelöst wird, werden die Gelenke 23, 34 und 45 frei, so daß hierdurch das rechte Ende der Halte- und Zieleinrichtung 10 mit der nach rechts weisenden Schwenkachse 6 höhenverstellbar ist und somit eine feinfühlige Verschwenkung um die nach links unten weisende Schwenkachse 6 ermöglicht wird.

Es sei darauf hingewiesen, daß hierbei der linke Haltearm arretiert ist, so daß die hier linke Schwenkachse 6 definiert bleibt. Somit wird hierdurch im wesentlichen eine Verschwenkung der Instrumentenspitze in Zeichenebene vorgenommen. Wenn jedoch die Verstellung quer zur Zeichenebene erfolgen soll, wird die rechte Arretierungsvorrichtung 12 fixiert und dafür die linke Arretierungsvorrichtung 12 gelöst. Somit ist eine Einstellung der Zieleinrichtung 10 in räumlich beliebiger Weise möglich. Insbesondere können hierbei durch Abknicken der Haltearme an dem Gelenk 34 zwischen dem Grundstab 3 und dem Haltestab 4 die Zieleinrichtung 10 abgesenkt werden, so daß diese möglichst nahe an dem Zielort Z gebracht werden kann, um somit Verbiegungen der sehr dünnen Punktionsnadeln zu vermeiden.

## Patentansprüche

1. Vorrichtung zur Ansteuerung körperlicher Strukturen, insbesondere zur Einführung von Punktionsnadeln oder Operationssonden,
- mit wenigstens einer Grundplatte (1),
- mit auf der Grundplatte (1) angebrachten Basishaltern (2),
- mit an den Basishaltern (2) gelenkig befestigten Haltestäben (3, 4),
- mit einer an den Haltestäben (3, 4) befestigten Halte- und Zieleinrichtung (10) für ein medizinisches Instrument (8), und
zwei winklig zueinander an der Halte- und Zieleinrichtung (10) angebrachten Schwenkachsen (6), die in Lagerelementen (5) verschwenkbar angeordnet sind,
dadurch gekennzeichnet, daß die Basishalter (2) auf der Grundplatte (1) lösbar anbringbar und beliebig positionierbar sind,
sich die Lagerelemente (5) am von der Grundplatte (1) abgewandten Ende der Haltestäbe (3, 4) befinden und die Schwenkachsen (6) sowohl verschwenkbar als auch verschiebbar angeordnet sind.

2. Vorrichtung nach Anspruch 1,
dadurch gekennzeichnet, daß
die mit den Basishaltern (2) verbundenen, frei schwenkbaren Haltestäbe (3, 4) jeweils mit einer zentralen Arretiervorrichtung (12) lagefixierbar sind.

3. Vorrichtung nach Anspruch 1 oder 2,
dadurch gekennzeichnet, daß
an der Grundplatte (1) und/oder den Basishaltern (2) komplementäre Formabschnitte (13), insbesondere Winkel, Stifte oder Schienen, zur Anbringung an stereotaktischen Vorrichtungen vorgesehen sind.

4. Vorrichtung nach einem der vorhergehenden Ansprüche,
dadurch gekennzeichnet, daß
die Grundplatte (1) an einem Operationstisch (1a) verstellbar anbringbar ist.

5. Vorrichtung nach einem der vorhergehenden Ansprüche,
dadurch gekennzeichnet, daß
die Grundplatte (1) Markierungen (14) zur Repositionierung der Basishalter (2) aufweist, die auf der Grundplatte (1) magnetisch, pneumatisch oder mechanisch verankerbar sind.

6. Vorrichtung nach einem der vorhergehenden Ansprüche,
dadurch gekennzeichnet, daß
an den Haltestäben (3, 4) Gelenke (23, 34, 45) angeordnet sind, die über Fluiddruck von der zentralen Arretiervorrichtung (12) aus lagefixier- und arretierbar sind.

7. Vorrichtung nach einem der vorhergehenden Ansprüche,
dadurch gekennzeichnet, daß
die Halte- und Zieleinrichtung (10) mit einer Vielzahl von Bohrungen (9) versehen ist, die vorzugsweise rasterförmig angeordnet sind.

8. Vorrichtung nach einem der vorhergehenden Ansprüche,
dadurch gekennzeichnet, daß
Einlagerohre (11) zur Anpassung an jeweilige Sonden- oder Nadeldurchmesser in den Bohrungen (9) fixierbar sind und daran Markierungen (16), insbesondere Längenskalen oder Numerierungen vorgesehen sind.

9. Vorrichtung nach einem der vorhergehenden Ansprüche,
dadurch gekennzeichnet, daß
das in eine Bohrung (9) der Halte- und Zieleinrichtung (10) eingeführte Instrument (8) nach Arretierung der Haltestäbe (4) in Zielposition (Z) durch eine Klemmeinrichtung (17) arretierbar ist.

10. Vorrichtung nach einem der vorhergehenden Ansprüche,
dadurch gekennzeichnet, daß
die Halte- und Zieleinrichtung (10) über einen Feinantrieb (15) mit den Haltestäben (4) zur Feineinstellung der Halte- und Zieleinrichtung (10) gegenüber den Haltestäben (3, 4) in wenigstens einem Freiheitsgrad (A, B) verbunden und in eingestellter Zielposition (Z) fixierbar ist.

11. Vorrichtung nach einem der vorhergehenden Ansprüche,
dadurch gekennzeichnet, daß
die Halte- und Zieleinrichtung (10) eine Würfelform aufweist und die Schwenkachsen (6) in Lagerblöcken (7) gelagert sind, die an der Halte- und Zieleinrichtung (10) befestigt sind.

## Claims

1. Device for controlling three-dimensional structures, in particular for inserting puncture needles or operation probes, comprising:
- at least one base plate (1),
- base holders (2) mounted on the base plate (1),
- holder rods (3, 4) secured in a hinged manner on the base holders (2),
- a holding and aiming arrangement (10) secured to the holder rods (3, 4) for a medical instrument (8), and
- two pivot shafts (6) mounted at an angle relative to each other on the holding and aiming arrangement (10) and pivotally disposed in bearing elements (5),
characterized in that
the base holders (2) can be detachably mounted in any position on the base plate (1), the bearing elements (5) are located at the distal ends of the holder rods (3, 4) with respect to the base plate (1), and the pivot shafts (6) are disposed so as to be both pivotable and displaceable.

2. Device according to claim 1, characterized in that the holder rods (3, 4) connected with the base holders (2) so as to be freely pivotable can each be locked in position by means of a central arresting means (12).

3. Device according to claim 1 or claim 2, characterized in that the base plate (1) and/or the base holders (2) are provided with complementary-shaped portions (13), in particular angles, pins, or rails, for their attachment to stereotactic apparatus.

4. Device according to any of the preceding claims, characterized in that the base plate (1) can be adjustably secured to an operating table (1a).

5. Device according to any of the preceding claims, characterized in that the base plate (1) includes markings (14) for repositioning the base holders (2) which can be tied to the base plate (1) in a magnetic, pneumatic, or mechanical way.

6. Device according to any of the preceding claims, characterized in that links (23, 34, 45) are positioned on the holder rods (3, 4), said links being adapted to be locked in position and arrested by means of fluid pressure from the central arresting means (12).

7. Device according to any of the preceding claims, characterized in that the holding and aiming arrangement (10) is provided with a plurality of bores (9) preferably aligned in a pattern-like manner.

8. Device according to any of the preceding claims, characterized in that liners (11) can be fixed in the bores (9) to adapt the latter to respective probe or needle diametres, and are provided with markings (16), in particular length scales or numberings.

9. Device according to any of the preceding claims, characterized in that the instrument (8) inserted in a bore (9) of the holding and aiming arrangement (10) can be locked in target position (Z) by a clamping means (17) following the arresting of the holder rods (4).

10. Device according to any of the preceding claims, characterized in that the holding and aiming arrangement (10) is connected with the holder rods (4) via a vernier drive (15) for fine adjustment of the holding and aiming arrangement (10) with respect to the holder rods (3, 4) in at least one degree of freedom (A, B), and can be fixed in the set target position (Z).

11. Device according to any of the preceding claims, characterized in that the holding and aiming arrangement (10) has a cubic shape and in that the pivot shafts (6) are pivoted in bearing blocks (7) which are secured to the holding and aiming arrangement (10).

## Revendications

1. Dispositif pour piloter des structures tridimensionnelles, en particulier pour l'introduction d'aiguilles de ponction ou de sondes d'opération,
- avec au moins un socle (1),
- avec des supports de base (2) disposés sur le socle (1),
- avec des tiges porteuses (3, 4) fixées de façon articulée aux supports de base (2),
- avec un dispositif de maintien et de pointage (10) destiné à un instrument médical (8) fixé aux tiges porteuses (3, 4), et
deux axes de pivotement (6) disposés sur le dispositif de maintien et de pointage (10) formant un angle l'un par rapport à l'autre, qui sont disposés de façon pivotante dans des éléments de palier (5),
caractérisé en en ce que les supports de base (2) peuvent être mis en place de façon amovible sur le socle (1) et sont positionnables à volonté, en ce que les éléments de palier (5) se trouvent à l'extrémité des tiges porteuses (3, 4) opposée au socle (1), et en ce que les axes de pivotement (6) sont disposés de façon à pouvoir être tant pivotés que déplacés.

2. Dispositif selon la revendication 1, caractérisé en ce que les tiges porteuses (3, 4), reliées aux supports de base (2) et pouvant pivoter librement, peuvent être fixées en position au moyen d'un dispositif de blocage central (12).

3. Dispositif selon la revendication 1 ou 2, caractérisé en ce que des éléments moulés complémentaires (13) sont prévus pour être raccordés au socle (1) et/ou aux supports de base (2), en particulier des équerres, des chevilles ou des rails, pour la mise en place à des dispositifs stéréotactiques.

4. Dispositif selon l'une quelconque des revendications précédentes, caractérisé en ce que le socle (1) peut se raccorder à une table d'opération (1a) de façon réglable.

5. Dispositif selon l'une quelconque des revendications précédentes, caractérisé en ce que le socle (1) comporte des repères (14) pour le repositionnement des supports de base (2), qui peuvent être ancrés sur le socle (1) de façon magnétique, pneumatique ou mécanique.

6. Dispositif selon l'une quelconque des revendications précédentes, caractérisé en ce que sur les tiges porteuses (3, 4) sont disposés des éléments articulés (23, 34, 45), dont la position peut être fixée et bloquée par le dispositif de blocage central (12) par pression de fluide.

7. Dispositif selon l'une quelconque des revendications précédentes, caractérisé en ce que le dispositif de maintien et de pointage (10) est pourvu d'une pluralité d'alésages (9), qui sont de préférence disposés en forme de quadrillage.

8. Dispositif selon l'une quelconque des revendications précédentes, caractérisé en ce que des tubes de logement (11), destinés à s'adapter aux diamètres respectifs de sondes ou d'aiguilles, peuvent être fixés dans les alésages (9) et en ce que des repères (16) figurent dessus, en particulier des échelles de longueur ou des numéros.

9. Dispositif selon l'une quelconque des revendications précédentes, caractérisé en ce que l'instrument (8) inséré dans un alésage (9) du dispositif de maintien et de pointage (10) peut être bloqué après blocage des tiges porteuses (4) en position de pointage (Z) par l'intermédiaire d'un dispositif de serrage (17).

10. Dispositif selon l'une quelconque des revendications précédentes, caractérisé en ce que le dispositif de maintien et de pointage (10) est relié aux tiges porteuses (4) avec au moins un degré de liberté (A, B) via une commande de précision (15) pour le réglage fin du dispositif de maintien et de pointage (10) par rapport aux tiges porteuses (3,4) et qu'il peut être fixé une fois sa position de pointage (Z) ajustée.

11. Dispositif selon l'une quelconque des revendications précédentes, caractérisé en ce que le dispositif de maintien et de pointage (10) présente une forme de cube et en ce que les axes de pivotement (6) sont logés dans des blocs de palier (7) qui sont fixés au dispositif de maintien et de pointage (10).
